# EUROPEAN PATENT APPLICATION

(11) **EP 4 145 328 A1**
(43) Date of publication of application: **08.03.2023**
(21) Application number: 21797897.2
(22) Date of filing: 09.04.2021
(51) Int. Cl.: G06F 30/10, G06F 30/27

(54) **SYSTEM THAT ESTIMATES CHARACTERISTIC VALUE OF MATERIAL**

(30) Priority: 28.04.2020 JP 2020079793
(71) Applicant: Hitachi, Ltd., Tokyo 100-8280 (JP)
(72) Inventor: KANAZAWA Takuya, Tokyo 100-8280 (JP); MORITA Hidekazu, Tokyo 100-8280 (JP); ASAHARA Akinori, Tokyo 100-8280 (JP); HAYASHI Takayuki, Tokyo 100-8280 (JP)
(74) Representative: MERH-IP Matias Erny Reichl Hoffmann Patentanwälte PartG mbB
(86) International application number: PCT/JP2021/015044
(87) International publication number: WO 2021/220776

(57) **Abstract**

One or more storage devices store a first machine learning model and a second machine learning model. The one or more processors generate each low-dimensional descriptor including the predetermined number of elements for multiple materials, and predict each characteristic value of the multiple materials from the low-dimensional descriptor. One or more processors select a part of materials from multiple materials based on the characteristic value, and generate a high-dimensional descriptor having the number of elements larger than the predetermined number. One or more processors predict each characteristic value of the part of the materials from the high-dimensional descriptor using the second machine learning model.

## Description

### Incorporation by reference

This application claims the benefit of foreign priority to Japanese Patent Application No. 2020-079793, filed on April 28, 2020, which is incorporated by reference in its entirety.

### Technical Field

The present invention relates to a system for predicting a material property value.

### Background Art

Conventionally, a virtual screening technique has been utilized for performing the task of new material search. The machine learning model is applied to data of known materials (compounds) to generate a characteristic value prediction model for predicting the material property value. More specifically, a descriptor indicating the material property expressed by a multivariate is generated from a chemical structure formula of the material. Furthermore, a relationship between the descriptor and the characteristic value is trained to generate the characteristic value prediction model. The characteristic value prediction model predicts the characteristic value in correspondence with the input descriptor. The descriptor includes multiple elements (feature values), indicating each characteristic of the respective elements, for example, a molecular weight, an element mixture ratio, and the like.

The virtual screening technique serves to generate the descriptor from chemical structure formulae of many compounds, each characteristic value of which is unknown. The characteristic value prediction model is applied to the above-described descriptors. The screening is executed based on the calculated characteristic value to present the chemical structure formula expected to have the characteristic value in excess of the threshold value as a prospective compound which becomes a candidate for an experiment or a simulation.

A user conducts the experiment or simulation of the materials selected from the candidates for evaluating those materials. Execution of the virtual screening reduces the required number of experiments and simulations of the material. This makes it possible to efficiently provide the material having the desired characteristic value.

The technique for generating the descriptor of the material has been disclosed in, for example, Non-Patent Literature 1 or Non-Patent Literature 2. They disclose the technique for finding out the descriptor constituted by combination of the small number of descriptor elements which are useful for prediction from several thousands to several tens of thousands of descriptor elements (feature values) in the inorganic chemistry field.

### Citation List

### NonPatent Literature

NPTL 1: L.M. Ghiringhelli et al., "Big Data of Materials Science: Critical Role of the Descriptor", Phys. Rev. Lett. 114, 105503 (2015)
NPTL 2: R. Ouyang et al., "SISSO: A compressed-sensing method for identifying the best low-dimensional descriptor in an immensity of offered candidates", Phys. Rev. Materials 2, 083802 (2018)

### Summary of Invention

### Technical Problem

In order to find out the compound having the desired characteristic value, the virtual screening is executed to generate descriptors of a vast amount of candidate compounds, and predict the characteristic value of the descriptor generated by the characteristic value prediction model. There are a large number of dimensions (the number of elements) of the descriptor which highly accurately expresses the characteristic of the chemical structure formula, generally, in the range from approximately 1000 to 4000.

Many computer resources and much time may be required for calculating all descriptors of the vast amount of candidate compounds. Most of the candidate compounds become inappropriate as they fail to provide the desired characteristic values. Calculation of the descriptors of those inappropriate candidate compounds may cause wasteful consumption of the computer resources and time.

Accordingly, it is desirable to provide the technique which allows efficient selection of a prospective material expected to have the desired characteristic value.

### Solution to Problem

According to an aspect of the present invention, a system for predicting the material property value includes one or more processors and one or more storage devices for storing programs to be executed by the one or more processors. The one or more storage devices store a first machine learning model and a second machine learning model. The one or more processors generate a low-dimensional descriptor including the predetermined number of elements for each of multiple materials. The one or more processors predict each characteristic value of the multiple materials from the low-dimensional descriptor using the first machine learning model. The one or more processors select a part of materials from the multiple materials based on the characteristic value. The one or more processors generate a high-dimensional descriptor having the number of elements larger than the predetermined number for each of the part of the materials. The one or more processors predict each characteristic value of the part of the materials from the high-dimensional descriptor using the second machine learning model.

### Advantageous Effects of Invention

According to the aspect of the present invention, it is possible to select the prospective material expected to have the desired characteristic value more efficiently.

### Brief Description of Drawings

Fig. 1 schematically illustrates a logical configuration example of a material property prediction apparatus according to an embodiment of the specification.
Fig. 2 illustrates a hardware configuration example of the material property prediction apparatus.
Fig. 3 is a flowchart representing an overall processing example of the material property prediction apparatus.
Fig. 4 schematically illustrates an example of a graphical user interface displayed on a monitor, through which material experimental data are input.
Fig. 5 illustrates a configuration example of an experimented material database.
Fig. 6 schematically illustrates an example of a graphical user interface displayed on the monitor, through which a list of material as a target for material property value prediction is input.
Fig. 7 illustrates a configuration example of a material formula database.
Fig. 8 is a flowchart representing a detailed learning processing operation applied to a low-dimensional material property prediction model.
Fig. 9 illustrates a configuration example of a descriptor list to be transmitted by the descriptor calculation module to the material property prediction model training module.
Fig. 10 illustrates an example of material property values (list of material property measured values) acquired by the material property prediction model training module from the experimented material database.
Fig. 11 illustrates a configuration example of material property prediction results (list of material property predicted values) to be transmitted from the material property prediction module to the material selection module.
Fig. 12 is a flowchart representing a detailed learning processing operation applied to a high-dimensional material property prediction model.
Fig. 13 illustrates an image example of the material property prediction results to be displayed on the monitor by a material property prediction result display module.

### Description of Embodiment

Hereinafter, an explanation will be made in multiple modules or embodiments separately for convenience as needed. Unless otherwise expressly specified, they are not irrelevant to one another. One of those modules or embodiments may constitute a modified example, detailed description, and complementary description as a part or all of the other. When making reference to the number of elements, and the like (including the number of elements, numerical value, amount, range, and the like), unless otherwise expressly or fundamentally limited to the specific number, such number is not limited thereto. It can be equal to, or either more or less than the specific number.

The system may be configured as a physical computer system (one or more physical computers), or a system constructed on a computer resource group (multiple computer resources) such as a cloud base. The computer system or the computer resource group includes one or more interface devices (including, for example, a communication device and an input/output device), one or more storage devices (including, for example, a memory (main storage) and an auxiliary storage device), and one or more processors.

In the case where the program is executed by the processor to implement the function, the prescribed processing is executed by appropriately using the storage device and/or the interface device, and the like. Accordingly, the function may be regarded as being at least a part of the processor. In the case where the function serves to execute the processing, the processing may be regarded as being executed by the processor or the system provided with the processor. The program may be installed from the program source. The program source may be a program distribution computer or a computer readable storage medium (for example, a computer readable non-fugitive storage medium). Explanations of the respective functions are mere examples. Multiple functions may be combined into a single function. Alternatively, a single function may be divided into multiple functions.

The following description discloses the technique that allows efficient selection of the material expected to have the desired material property in the virtual screening. The material property prediction apparatus executes two-stage refinement processing to the population of candidate materials.

Specifically, the material property prediction apparatus calculates the respective low-dimensional descriptors for all the candidate materials. The material property prediction apparatus predicts each material property value from the respective low-dimensional descriptors using the simple machine learning model. The material property prediction apparatus selects a part of the materials based on the material property predicted values.

The material property prediction apparatus calculates the respective high-dimensional descriptors for the selected materials. The material property prediction apparatus predicts each material property value from the respective high-dimensional descriptors using the machine learning model with hither accuracy. Based on those material property predicted values, the material property prediction apparatus selects the material to be presented to the user as the final candidate. As described above, the material for generation of the high-dimensional descriptor is selected based on the material property prediction results from the low-dimensional descriptors. This makes it possible to efficiently select the material expected to have the desired material property at high speeds.

An embodiment of the specification will be described in more detail. An explanation will be made with respect to an example of prediction of the characteristic value of an organic compound, which is expressed in the chemical structure formula. In the specification, the material property prediction apparatus may be applicable both to the organic compound and the inorganic compound. The descriptor may be generated from the chemical formula, that is, either a structural formula or a compositional formula.

### First Embodiment

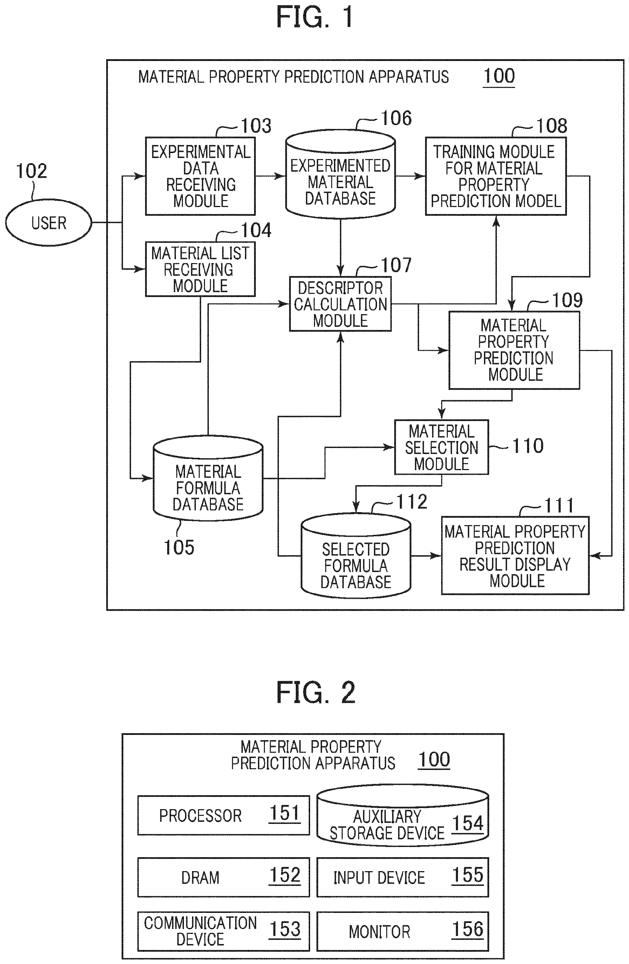

Fig. 1 schematically illustrates a logical configuration example of the material property prediction apparatus according to an embodiment of the present specification. A material property prediction apparatus 100 stores a material formula database 105, an experimented material database 106, and a selected formula database 112.

The material property prediction apparatus 100 includes an experimental data reception module 103, a material list reception module 104, a descriptor calculation module 107, a material property prediction model training module 108, a material property prediction module 109, a material selection module 110, and a material property prediction result display module 111, all of which are programs. One or more processors of the material property prediction apparatus 100 serve as corresponding function modules by executing those programs. An arbitrary function of the material property prediction apparatus 100 may be implemented in an arbitrary program.

The experimental data reception module 103 receives experimental data indicating characteristic values of various materials, which have been input by a user 102 through the input/output device, and stores the data in the experimented material database 106. The material list reception module 104 receives chemical structure formula data of various materials, which have been input by the user 102 through the input/output device, and stores the data in the material formula database 105. The material formula database 105 stores data of materials (chemical structure formulae) which are not stored in the experimented material database 106.

The descriptor calculation module 107 generates a descriptor from the chemical structure formula using a predetermined method. The descriptor indicates a characteristic of the material expressed by the chemical structure formula. The descriptor is expressed by a vector constituted by multiple elements (feature values). The characteristic corresponding to each element represents, for example, a molecular weight and an element mixing ratio. As described later, the descriptor calculation module 107 is capable of generating a low-dimensional descriptor having a small number of elements, and a high-dimensional descriptor having a large number of elements from the single chemical structure formula. The descriptor calculation module 107 may be divided into modules for generating the low-dimensional descriptor and the high-dimensional descriptor, respectively.

Each number and each type of the elements of the low-dimensional descriptor and the high-dimensional descriptor are kept constant. The number of elements of the low-dimensional descriptor is smaller than the number of elements of the high-dimensional descriptor. All types of the elements of the low-dimensional descriptor may be included in the types of elements of the high-dimensional descriptor. The elements of the low-dimensional descriptor may be partially or entirely different from elements of the high-dimensional descriptor in type.

In the case where all types of elements of the low-dimensional descriptor are included in the types of elements of the high-dimensional descriptor, it is possible to determine more appropriate type of the element of the low-dimensional descriptor from a viewpoint of the machine learning model for predicting the characteristic value from the high-dimensional descriptor. The descriptor calculation module 107 may be configured to determine importance placed on prediction of the characteristic value of the element of the high-dimensional descriptor, and further to determine the elements constituting the low-dimensional descriptor based on the importance. Prediction of the material property value from the low-dimensional descriptor allows selection of more appropriate candidate material.

For example, the descriptor calculation module 107 is configured to execute learning by means of a decision tree base ensemble learner such as a random forest and a gradient boosting using the high-dimensional descriptor, and to calculate the importance placed on each element of the high-dimensional descriptor. The descriptor calculation module 107 selects the predetermined number of descriptors from the element with the highest importance.

In another example, the descriptor calculation module 107 may be configured to determine the importance placed on the element of the high-dimensional descriptor using linear regression by Permutation Importance, LASSO, and the like. The machine learning model for selecting the element of the low-dimensional descriptor may be the same as or different from the machine learning model for predicting the material property value from the high-dimensional descriptor. The algorithms for those models may be the same or different from one another.

The material property prediction model training module 108 executes learning of a material property prediction model (machine learning model) which is capable of predicting a predetermined characteristic value from the descriptor of the chemical structure formula (material). As described later, the material property prediction apparatus 100 according to the embodiment of the specification provides a low-dimensional material property prediction model (first machine learning model) and a high-dimensional material property prediction model (second machine learning model). The first machine learning model predicts one or more predetermined types of characteristic values from the low-dimensional descriptor. The second machine learning model predicts the similar types of characteristic values from the high-dimensional descriptor.

The configuration may be designed to provide multiple low-dimensional material property prediction models. Each number of dimensions of the models may be different from or common to one another. Combinations of element types among those models may be the same or different from one another. Every number of dimensions of the low-dimensional descriptors of the low-dimensional material property prediction models is smaller than the number of dimensions of the high-dimensional descriptor.

The material property prediction model may be configured to predict one or more types of characteristic values. In the following example, it is assumed that the material property prediction model predicts (outputs) a single characteristic value. Arbitrary regression algorithms may be utilized by the low-dimensional material property prediction model and the high-dimensional material property prediction model. Those algorithms may be the same or different from each another. An arbitrary algorithm may be selected from various types of regression algorithms including the random forest, support vector machine, Gaussian process regression, and neural network.

The material property prediction module 109 uses the trained low-dimensional material property prediction model to obtain an predicted material property value from the low-dimensional descriptor, and further uses the trained high-dimensional material property prediction model to obtain an predicted material property value from the high-dimensional descriptor. In an example to be described below, the low-dimensional descriptor is generated from all chemical structure formulae (materials) stored in the material formula database 105. The high-dimensional descriptor is generated with respect to the material corresponding to the low-dimensional descriptor having the predicted material property value approximate to an ideal value.

The material selection module 110 selects the material (chemical structure formula) for generation of the high-dimensional descriptor based on the material property value predicted with respect to the low-dimensional descriptor, and stores the information (chemical structure formula) in the selected formula database 112. The criteria of material selection depends on the nature of the material property value, and a requirement of the user. It is possible to select the predetermined number of materials, each having the characteristic predicted value of the material property value approximate to the target value, or the material included in a predetermined range.

In the case where the higher material property value is preferable, the material selection module 110 may be configured to select the predetermined number of materials, each having the highest material property value, or the material having the material property value in excess of a predetermined threshold value. In the case where the lower material property value is preferable, the material selection module 110 may be configured to select the predetermined number of materials, each having the lowest material property value, or the material having the material property value smaller than the predetermined threshold value.

In the case where multiple low-dimensional material property prediction models are provided, the material for generation of the high-dimensional descriptor may be selected based on a statistic of predicted values of those multiple low-dimensional material property prediction models (for example, weighted mean value (including mean value)).

The material property prediction result display module 111 acquires a material property value prediction result from the high-dimensional descriptor of the selected material using the high-dimensional material property value prediction model. The material property prediction result display module 111 displays the material property value prediction result together with the corresponding chemical structure to present the prospective material to the user 102. The material property prediction result display module 111 may be configured to display the prediction results of all the selected materials, or only a part of the materials each indicating the preferable predicted value, which have been selected based on the predetermined criteria.

Fig. 2 illustrates a hardware configuration example of the material property prediction apparatus 100. The material property prediction apparatus 100 includes a processor 151 which performs calculation operations, and a DRAM 152 for providing a volatile temporary storage region which stores programs to be executed by the processor 151, and data. The material property prediction apparatus 100 further includes a communication device 153 for executing data communication with other devices, and an auxiliary storage device 154 for providing a persistent information storage region using an HDD (Hard Disk Drive), a flash memory, and the like.

For example, the auxiliary storage device 154 stores programs corresponding to the experimental data reception module 103, the material list reception module 104, the descriptor calculation module 107, the material property prediction model training module 108, the material property prediction module 109, the material selection module 110, the material property prediction result display module 111, and the like. The auxiliary storage device 154 further stores the respective data in the material formula database 105, the experimented material database 106, the selected formula database 112, and the like. The programs to be executed by the processor 151, and the data to be processed are loaded from the auxiliary storage device 154 to the DRAM 152.

The material property prediction apparatus 100 includes an input device 155 for receiving operations from the user, and a monitor (exemplified by an output device) 156 for displaying output results of the respective processing operations to the user. Functions of the material property prediction apparatus 100 may be divided into multiple devices to be implemented. As described above, the material property prediction apparatus 100 includes one or more storage devices, and one or more processors.

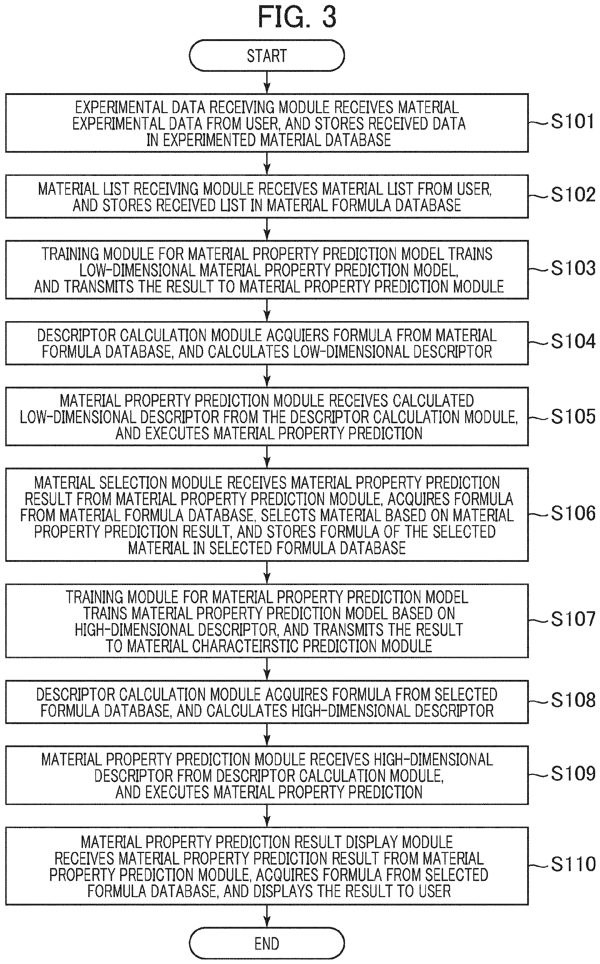

Fig. 3 is a flowchart representing an example of overall processing executed in the material property prediction apparatus 100. In step S101, the experimental data reception module 103 receives material experimental data from the user 102 through the input device 155, and stores the data in the experimented material database 106. In step S102, the material list reception module 104 receives a material list from the user 102 through the input device 155, and stores the material list in the material formula database 105.

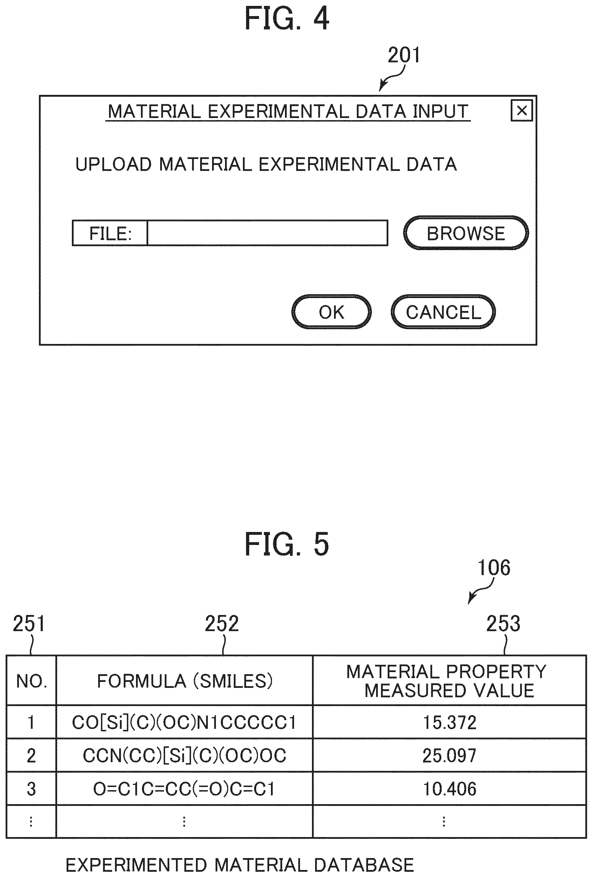

Fig. 4 schematically illustrates an example of a graphical user interface (GUI) 201 displayed on the monitor 156, through which the material experimental data are input. The user inputs necessary information to the GUI 201 through the input device 155. The user designates a file which stores the experimental data using a "browse button" on the GUI 201, and selects an "OK" button to instruct the experimental data reception module 103 to receive the file. The experimental data reception module 103 stores data of the designated file in the experimented material database 106.

Fig. 5 illustrates a configuration example of the experimented material database 106. The experimented material database 106 makes the material in correspondence with an experimental result of the characteristic value of the material. Specifically, the experimented material database 106 is composed of a number column 251, a formula (SMILES) column 252, and a material property measured value column 253.

The number column 251 identifies each record in the experimented material database 106. The formula (SMILES) column 252 provides each chemical structure formula of the materials. Referring to the example of Fig. 4, the chemical structure formula is expressed in accordance with a notation of SMILES (Simplified Molecular Input Line Entry System). The chemical structure formula may be arbitrarily represented to generate the descriptor. The material property measured value column 253 represents each experimental result of predetermined characteristic values of the chemical structure formulae. The measured values stored in the experimented material database 106 (measurement database) may be the measured values of simulation results either partially or entirely.

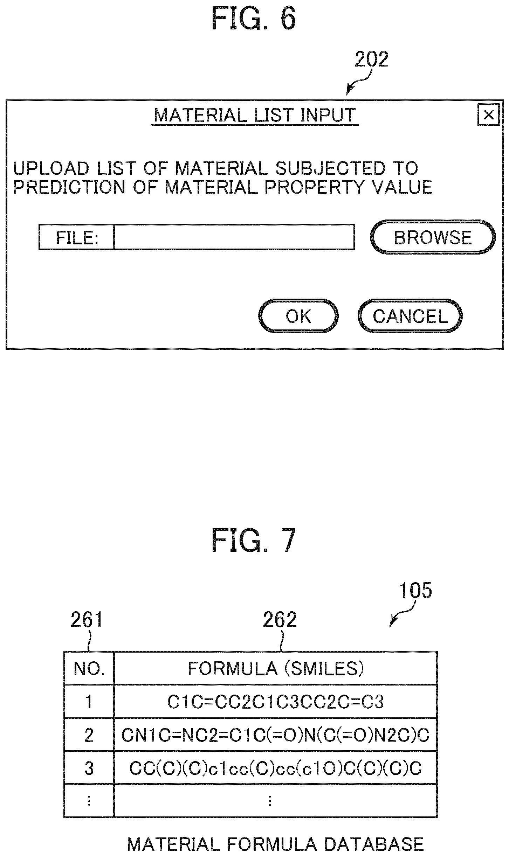

Fig. 6 schematically illustrates an example of a GUI 202 displayed on the monitor 156, through which the list of materials for material property value prediction is input. The user inputs necessary information to the GUI 202 through the input device 155. The user designates a file which stores the material list using a "browse button" to the GUI 202, and selects an "OK" button to instruct the material list reception module 104 to receive the file. The material list reception module 104 stores data of the designated file in the material formula database 105.

Fig. 7 illustrates a configuration example of the material formula database 105. The material formula database 105 stores chemical structure formulae to be subjected to material property value prediction. In the example to be described herein, the low-dimensional descriptors of all materials stored in the material formula database 105 are generated so that the respective material property values are predicted. In another example, the materials (chemical structure formulae) as a part of those stored in the material formula database 105 may be selected for generating the low-dimensional descriptors. For example, the predetermined number of materials may be randomly selected.

Referring to the example of Fig. 7, the material formula database 105 is composed of a number column 261, and a formula (SMILES) column 262. The number column 261 identifies each record in the material formula database 105. The formula (SMILES) column 262 represents SMILES expression of each chemical structure formula of materials.

Referring back to Fig. 3, in step S103, the material property prediction model training module 108 executes training of the low-dimensional material property prediction model, and transmits the model to the trained material property prediction module 109. In this case, it is assumed that the single low-dimensional material property prediction model is formed.

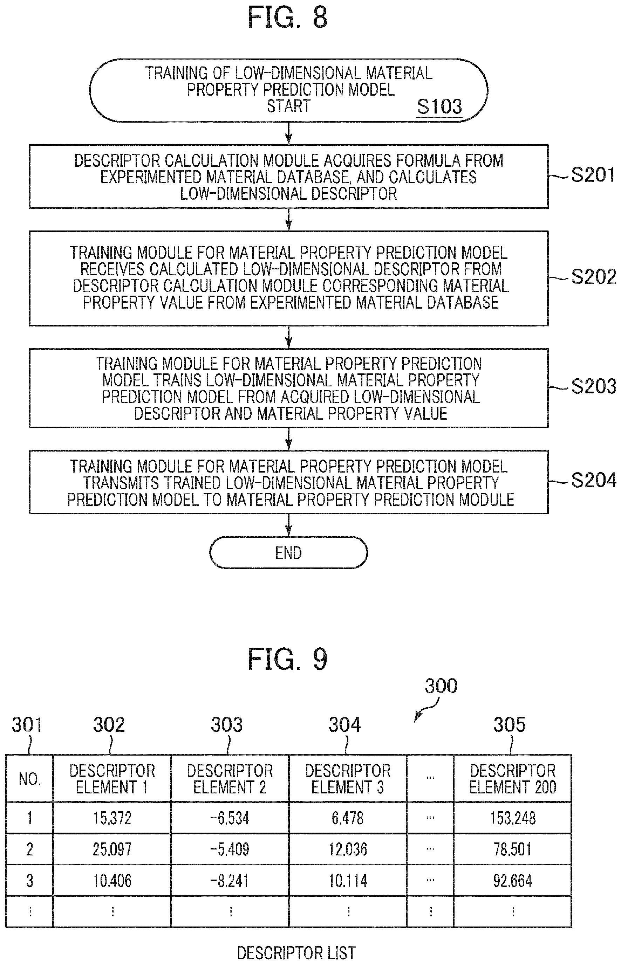

Fig. 8 is a flowchart representing the detailed training processing (S103) of the low-dimensional material property prediction model. In step S201, in response to an instruction of the material property prediction model training module 108, the descriptor calculation module 107 acquires materials (chemical structure formulae) partially or entirely from the experimented material database 106, and calculates the respective low-dimensional descriptors.

In an exemplary case, the number of materials (training data quantity) acquired from the experimented material database 106 for training of the low-dimensional material property prediction model is smaller than the number of materials (training data quantity) acquired for the high-dimensional material property prediction model to be described later. The number of dimensions of the low-dimensional material property prediction model is smaller than that of the high-dimensional material property prediction model. Therefore, it is possible to execute efficient and appropriate training using training data smaller in size than those for the high-dimensional material property prediction model.

The data acquired from the experimented material database 106 indicate values both in the number column 251 and the formula column 252 of the experimented material database 106. The type and the number of the descriptor elements constituting the low-dimensional descriptor are preliminarily set in the apparatus. Alternatively, they are set through selection from elements of the high-dimensional descriptor based on the importance.

In step S202, the material property prediction model training module 108 receives the calculated low-dimensional descriptor from the descriptor calculation module 107, and acquires the material property value (list of material property measured values) of chemical structure formula corresponding to the calculated low-dimensional descriptor from the experimented material database 106.

Fig. 9 illustrates a configuration example of the descriptor list to be transmitted to the material property prediction model training module 108 by the descriptor calculation module 107. Fig. 9 illustrates an example of a descriptor list 300 of the low-dimensional descriptor to be transmitted by the descriptor calculation module 107. The table configuration of the high-dimensional descriptor list is similar to the illustrated one except that the number of elements of the descriptor is smaller.

The descriptor list 300 is composed of a number column 301, and columns of respective descriptor elements. Values in the number column 301 correspond to those in the number column 251 of the experimented material database 106. In the experimental data example illustrated in Fig. 9, the descriptor is constituted by 4000 descriptor elements. The example shows four columns of descriptor elements, designated with codes 302 to 305.

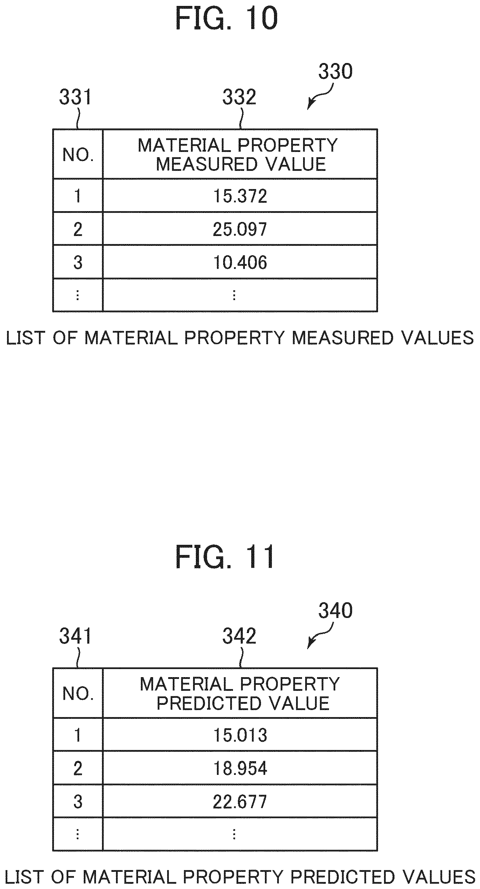

Fig. 10 illustrates an example of material property measured values (list of material property measured values) 330, which have been acquired by the material property prediction model training module 108 from the experimented material database 106. The list of material property measured values is composed of a number column 331 and a material property measured value column 332. Values in the number column 331 correspond to those in the number column 251 of the experimented material database 106. Values in the material property measured value column 332 correspond to those in the material property measured value column 253.

Referring back to Fig. 8, in step S203, the material property prediction model training module 108 executes training of the low-dimensional material property prediction model from the acquired low-dimensional descriptor and the material property value. The material property prediction model training module 108 preliminarily stores information on an initial configuration of the low-dimensional material property prediction model, based on which the low-dimensional material property prediction model is formed. As described above, the machine learning model of arbitrary type may be used for the low-dimensional material property prediction model.

The material property prediction model training module 108 inputs the low-dimensional descriptors to the low-dimensional material property prediction model sequentially, and acquires output predicted values of the material property values. The material property prediction model training module 108 updates a parameter of the low-dimensional material property prediction model based on an error between the predicted value of the material property value and the acquired material property measured value so that the low-dimensional material property prediction model is optimized. Finally, in step S204, the material property prediction model training module 108 transmits the trained low-dimensional material property prediction model to the material property prediction module 109.

Referring back to Fig. 3, in step S104, in response to an instruction from the material property prediction module 109, the descriptor calculation module 107 acquires the chemical structure formulae (records) partially or entirely from the material formula database 105, and calculates each of the low-dimensional descriptors. In the data acquired by the descriptor calculation module 107 from the material formula database 105, correspondence between the number and the chemical structure formula is similar to the correspondence in the material formula database 105 as shown in Fig. 7.

In step S105, the material property prediction module 109 receives the calculated low-dimensional descriptors from the descriptor calculation module 107, and executes material property prediction. Specifically, the material property prediction module 109 inputs each of the acquired low-dimensional descriptors to the trained low-dimensional material property prediction model, and acquires the corresponding characteristic predicted values.

In step S106, the material selection module 110 receives a material property prediction result from the material property prediction module 109, and acquires a chemical structure formula with the number indicated by the received prediction result from the material formula database 105. The material selection module 110 selects the material (chemical structure formula) based on the material property prediction result, and stores the selected chemical structure formula in the selected formula database 112. The data configuration of the selected formula database 112 may be similar to that of the material formula database 105 as well as the number in correspondence with the chemical structure formula.

Fig. 11 illustrates a configuration example of a material property prediction results (list of material property predicted values) 340, which are transmitted from the material property prediction module 109 to the material selection module 110. The list of material property predicted values 340 is composed of a number column 341 and a material property predicted value column 342. Values in the number column 341 correspond to those in the number column 261 of the material formula database 105. Values in the material property predicted value column 342 indicate material property predicted values of the chemical structure formulae with the respective numbers in the number column 341.

The material selection module 110 selects the material having the characteristic predicted value that conforms to a predetermined condition with reference to the list of material property predicted values 340, and stores the chemical structure formula of the selected material in the selected formula database 112.

Referring back to Fig. 3, in step S107, the material property prediction model training module 108 executes training of the high-dimensional material property prediction model, and transmits the trained high-dimensional material property prediction model to the material property prediction module 109.

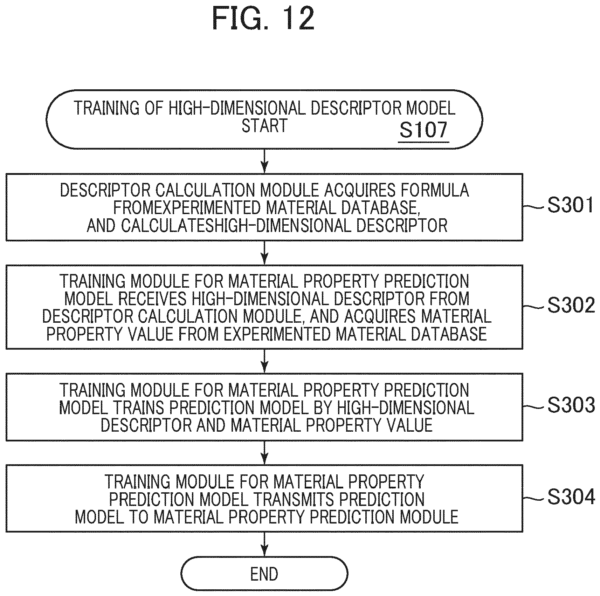

Fig. 12 is a flowchart representing detailed training processing (S107) of the high-dimensional material property prediction model. In step S301, in response to an instruction of the material property prediction model training module 108, the descriptor calculation module 107 acquires the materials (chemical structure formulae) partially or entirely from the experimented material database 106, and calculates the respective high-dimensional descriptors. The data acquired from the experimented material database 106 indicate values both in the number column 251 and the formula column 252 of the experimented material database 106. The type and the number of the descriptor elements constituting the high-dimensional descriptor are preliminarily set.

Then in step S302, the material property prediction model training module 108 receives the calculated high-dimensional descriptors (descriptor list) from the descriptor calculation module 107. The configuration of the descriptor list to be transmitted by the descriptor calculation module 107 to the material property prediction model training module 108 is similar to that of the descriptor list as illustrated in Fig. 9. The records of those lists may be the same or different from one another.

The material property prediction model training module 108 acquires material property values of the chemical structure formulae (list of material property measured values) corresponding to the calculated high-dimensional descriptors from the experimented material database 106. The configuration of the list of material property measured values is similar to that of the list of material property measured values 330 as shown in Fig. 10. The number in the list of material property measured values (corresponding material) matches the number (corresponding material) in the high-dimensional descriptor list.

In step S303, the material property prediction model training module 108 executes training of the high-dimensional material property prediction model from the acquired high-dimensional descriptor and the material property value. The material property prediction model training module 108 preliminarily stores information on an initial configuration of the high-dimensional characteristic prediction model, based on which the high-dimensional material property prediction model is formed. As described above, the machine learning model of arbitrary type may be used for the high-dimensional material property prediction model.

The material property prediction model training module 108 inputs the high-dimensional descriptors to the high-dimensional material property prediction model sequentially, and acquires output predicted values of the material property values. The material property prediction model training module 108 updates a parameter of the high-dimensional material property prediction model based on an error between the predicted value of the material property value and the acquired material property measured value so that the high-dimensional material property prediction model is optimized. Finally, in step S304, the material property prediction model training module 108 transmits the trained high-dimensional material property prediction model to the material property prediction module 109.

Referring back to Fig. 3, in step S108, in response to an instruction from the material property prediction module 109, the descriptor calculation module 107 acquires the chemical structure formulae from the selected formula database 112, and calculates each of the high-dimensional descriptors. Calculation is performed with respect to the high-dimensional descriptor only of the material selected based on the characteristic predicted value from the low-dimensional descriptor. This makes it possible to perform calculation of the high-dimensional descriptor and subsequent calculation of the characteristic predicted value at high speeds.

In step S109, the material property prediction module 109 receives the high-dimensional descriptors of the chemical structure formulae stored in the selected formula database 112 from the descriptor calculation module 107, and executes material property prediction. Specifically, the material property prediction module 109 inputs each of the acquired high-dimensional descriptors to the trained high-dimensional material property prediction model sequentially. The high-dimensional material property prediction model outputs each material property predicted value of the input high-dimensional descriptors, respectively.

In step S110, the material property prediction result display module 111 receives a material property prediction result of the selected chemical structure formula from the material property prediction module 109. The material property prediction result display module 111 further acquires the chemical structure formula from the selected formula database 112. The material property prediction result display module 111 displays the acquired material property prediction result and the chemical structure formula to the user.

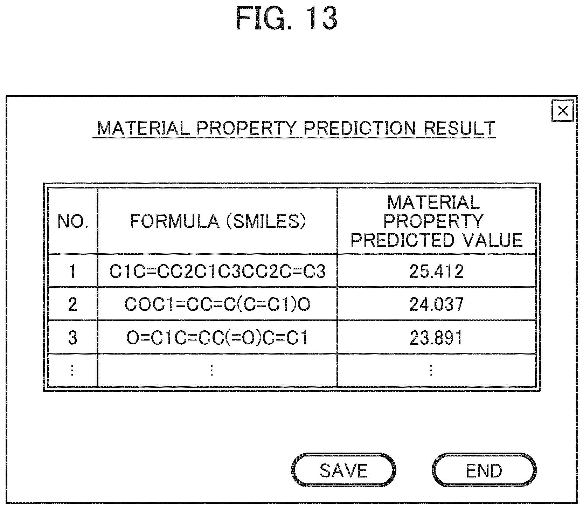

Fig. 13 illustrates an image example of the material property prediction result to be displayed on the monitor 156 by the material property prediction result display module 111. Referring to the example of Fig. 13, the images represent the chemical structure formulae of the selected materials, and predicted values of the corresponding material property values. With reference to the displayed chemical structure formulae and the material property values, the user is allowed to determine the chemical structure formula for actual execution of the experiment or simulation. The prediction result is stored through a save button.

The present invention is not limited to the embodiment as described above, but includes various modifications. For example, the embodiment is described in detail for readily understanding of the present invention which is not necessarily limited to the one equipped with all structures as described above. It is possible to replace a part of the structure of one embodiment with the structure of another embodiment. The one embodiment may be provided with an additional structure of another embodiment. It is further possible to add, remove, and replace the other structure to, from and with a part of the structure of the respective embodiments.

The respective structures, functions, processing parts, and the like may be realized through hardware by designing those elements partially or entirely using the integrated circuit, for example. The respective structures and functions may also be realized through software by interpreting and executing the program for the processor to implement the respective functions. Information on the program, table, file, and the like for implementing the respective functions may be stored in the storage unit such as a memory, a hard disk, an SSD (Solid State Drive), or a recording medium such as an IC card and an SD card.

The control line and information line considered as necessary for explanations are only shown. They do not necessarily represent all the control and information lines for the product. Actually, it may be considered that almost all the components are connected with one another.

## Claims

1. A system for predicting a material property value, the system including one or more processors and one or more storage devices, wherein:
the one or more storage devices store a first machine learning model and a second machine learning model; and
the one or more processors generate a low-dimensional descriptor including the predetermined number of elements for each of multiple materials, predict each of the characteristic values of the multiple materials from the low-dimensional descriptor using the first machine learning model, select a part of materials from the multiple materials based on the characteristic value, generate a high-dimensional descriptor having the number of elements larger than the predetermined number for each of the part of the materials, and predict each of the characteristic values of the part of the materials from the high-dimensional descriptor using the second machine learning model.

2. The system according to claim 1, wherein types of all elements of the low-dimensional descriptor are included in types of elements of the high-dimensional descriptor.

3. The system according to claim 1, wherein:
the one or more processors calculate importance to be placed on characteristic value prediction of the type of the element of the high-dimensional descriptor by implementing a prescribed method, and select the type of the element of the low-dimensional descriptor from the type of the element of the high-dimensional descriptor based on the importance.

4. The system according to claim 1, wherein:
the storage device stores measurement data including characteristic measured values of the multiple materials; and
the one or more processors execute training of the first machine learning model using first training data selected from the measurement data, and learning of the second machine learning model using second training data larger in size than the first training data selected from the measurement data.

5. The system according to claim 1, wherein the one or more processors output the part of the materials, and the characteristic value predicted by the second machine learning model on a monitor.

6. A method of predicting a material property value, the method being implemented by a system including one or more processors and one or more storage devices, and the one or more devices storing a first machine learning model and a second machine learning model, wherein:
the one or more processors generate a low-dimensional descriptor including the predetermined number of elements for each of multiple materials;
the one or more processors predict each of the characteristic values of the multiple materials from the low-dimensional descriptor using the first machine learning model;
the one or more processors select a part of materials from the multiple materials based on the characteristic values;
the one or more processors generate a high-dimensional descriptor having the number of elements larger than the predetermined number for each of the part of the materials; and
the one or more processors predict each of the characteristic values of the part of the materials from the high-dimensional descriptor using the second machine learning model.

7. The method according to claim 6, wherein types of all elements of the low-dimensional descriptor are included in types of elements of the high-dimensional descriptor.

8. The method according to claim 6, wherein:
the one or more processors calculate importance to be placed on characteristic value prediction of the type of the element of the high-dimensional descriptor, and select the type of the element of the low-dimensional descriptor from the type of the element of the high-dimensional descriptor based on the importance.

9. The method according to claim 6, wherein:
the storage device stores measurement data including characteristic measured values of the multiple materials;
the method allows the one or more processors to execute learning of the first machine learning model using first training data selected from the measurement data, and learning of the second machine learning model using second training data larger in size than the first training data selected from the measurement data.

10. The method according to claim 6, wherein the one or more processors output the part of the materials, and the characteristic value predicted by the second machine learning model on a monitor.
